# EUROPÄISCHE PATENTSCHRIFT

(11) **EP 2 486 883 B1**
(45) Veröffentlichungstag und Bekanntmachung des Hinweises auf die Patenterteilung: **23.04.2014**
(21) Anmeldenummer: 11154266.8
(22) Anmeldetag: 14.02.2011
(51) Int. Cl.: A61B 18/12, A61B 17/3203, A61B 18/02

(54) **Versorgungseinrichtung**
Supply device
Dispositif d'alimentation

(43) Veröffentlichungstag der Anmeldung: 15.08.2012
(73) Patentinhaber: ERBE Elektromedizin GmbH, 72072 Tübingen (DE)
(72) Erfinder: Fritz, Martin, 72070 Tübingen (DE)
(74) Vertreter: Bohnenberger, Johannes

(56) Entgegenhaltungen:
- WO-A1-96/13216
- US-A1- 2007 179 495
- US-A1- 2009 149 917
- US-A1- 2010 125 292

## Beschreibung

Die Erfindung betrifft eine Versorgungseinrichtung für ein Chirurgiegerät zum Betreiben mindestens eines chirurgischen Instruments gemäß dem Oberbegriff des Anspruchs 1 sowie ein Verfahren zur Erzeugung eines Steuerprogramms für eine Versorgungseinrichtung gemäß dem Oberbegriff des Anspruchs 8.

Eine Versorgungseinrichtung der hier angesprochenen Art ist beispielsweise aus der DE 10 2009 042 428, US 2009/149917 A1 und WO 96/13216 A1 bekannt. Die Versorgungseinrichtung dient zum Betreiben mindestens eines chirurgischen Instruments, insbesondere eines elektrochirurgischen, kryochirurgischen oder eines wasserstrahlchirurgischen Instruments. Sie umfasst eine Steuereinheit zur Steuerung des mindestens einen Instruments sowie eine Speichereinheit zum Speichern von Konfigurationsdaten, die einen endlichen Automaten, nämlich einen Zustandsautomaten mit einer Vielzahl von Zuständen beschreibt. Die Steuereinheit ist derart ausgebildet, dass sie die Konfigurationsdaten einliest, den endlichen Automaten in ein Steuerprogramm übersetzt und das mindestens eine Instrument gemäß dem Steuerprogramm steuert. Ein chirurgisches Instrument, welches an ein Chirurgiegerät angeschlossen ist, kann auf diese Weise einfach programmiert werden und eine Prüfung der Korrektheit der Programmierung ist durch die Anwendung eines Zustandsautomaten schnell und effizient gewährleistet. Bei der bekannten Versorgungseinrichtung wird ein Rahmenprogramm oder ein Framework innerhalb der Versorgungseinrichtung vorgesehen, welches das Einlesen und Umsetzen eines Steuerprogramms für die Instrumente in Form eines Zustandsautomaten ermöglicht. Der Begriff "Zustandsautomat" wird im Folgenden als synonymer Begriff zu einem endlichen Automaten bzw. einer Zustandsmaschine verwendet, d.h. also allgemein ein Modell eines Verhaltens, bestehend aus Zuständen, Zustandsübergängen und Aktionen. Das oben angesprochene Rahmenprogramm muss üblicherweise nicht verändert werden, um die Funktionsweise der Versorgungseinrichtung weiter zu entwickeln. Die eigentliche Steuerung der Instrumente erfolgt anhand des Zustandsautomaten, der durch die Konfigurationsdaten beschrieben wird. Die Korrektheit eines Zustandsautomaten lässt sich sehr einfach validieren. Es werden vorzugsweise deterministische Automaten verwendet, so dass einfach überprüft werden kann, ob der Automat korrekt arbeitet. Aufgrund der Verwendung von Zustandsautomaten lässt sich der Arbeitsaufwand bei der Entwicklung von neuen Steuer- und Regelalgorithmen minimieren, wobei stets die Sicherheit des Patienten und des die Versorgungseinrichtung bedienenden Personals gewährleistet wird.

Bei den Versorgungseinrichtungen der bekannten Art ergibt sich das Problem, dass während des Ablaufs des Steuerprogramms, beispielsweise zur Veränderung der Intensität eines Gewebeeffekts und insbesondere zur Veränderung der Spannung, eines Stroms oder einer Leistung, unter Umständen an mehreren Stellen im Ablauf des Zustandsautomaten eine Veränderung der Vorgabewerte bzw. der Stellwerte vorgenommen werden muss. Bei herkömmlichen Versorgungseinrichtungen nimmt in diesem Fall ein Benutzer manuelle Einstellungen an Einstelleinrichtungen wie Potentiometern oder dergleichen vor. Eine derartige manuelle Einstellung von Parametern ist jedoch bei Verwendung eines Zustandsautomaten aufgrund der Komplexität des Systems nicht mehr möglich. Während des Ablaufs des Zustandsautomaten kann ein Benutzer durch Betätigung einer Einstelleinrichtung des Chirurgiegeräts folglich keine manuelle Veränderung beispielsweise der Ausgangsspannung oder einer Ausgangsleistung oder dergleichen vornehmen. Zwar ist es denkbar, für jeden möglichen Einstellwert der Einstelleinrichtung(en) eine eigene Steuertabelle für den Zustandsautomaten zu generieren, was jedoch mit einem enormen Aufwand verbunden ist und die Vorteile der Übersichtlichkeit und einfachen Wartung der Steuerung mittels eines Zustandsautomaten stark vermindern würde.

Ein weiterer Nachteil der bekannten Versorgungseinrichtung ist es, dass Gewebeparameter und/oder HF-Messwerte, welche die Zustandsübergänge des Automaten beeinflussen, nur den Ablauf des Zustandsautomaten bestimmen und nicht die Intensität einzelner Stellwerte. Dabei wäre es wünschenswert, dass Gewebeparameter und/oder HF-Messwerte eben nicht nur den Ablauf des Zustandsautomaten beeinflussen, sondern darüber hinaus eine Veränderung von Stellwerten, wie beispielsweise die Spannung, Zeit, Strom, Leistung usw., bewirken können.

Aufgabe der vorliegenden Erfindung ist es daher, eine Versorgungseinrichtung für ein Chirurgiegerät zum Betreiben mindestens eines chirurgischen Instruments zu schaffen, die einerseits eine manuelle Einflussnahme durch einen Benutzer auf Stellwerte des Zustandsautomaten während des Ablaufs des Zustandsautomaten zulässt und die andererseits eine Veränderung von Stellwerten durch Gewebeparameter und/oder HF-Messwerte oder dergleichen zulässt, welche als Bedingungen für Zustandsübergänge zwischen zwei Zuständen des Zustandsautomaten herangezogen werden.

Zur Lösung der oben genannten Aufgabe wird eine Versorgungseinrichtung mit den Merkmalen des Anspruchs 1 vorgeschlagen. Die Versorgungseinrichtung für ein Chirurgiegerät zum Betreiben mindestens eines chirurgischen Instruments, insbesondere eines elektrochirurgischen Instruments und/oder eines kryochirurgischen Instruments und/oder wasserstrahlchirurgischen Instruments, umfasst eine Steuereinheit zum Steuern des mindestens einen Instruments, sowie eine Speichereinheit zum Speichern von Konfigurationsdaten, insbesondere Stellwerte wie HF-Spannung, Leistung, Zeitspannen oder dergleichen, die einen Zustandsautomaten mit einer Vielzahl von Zuständen beschreiben. Dabei ist die Steuereinheit so ausgebildet, dass sie die Konfigurationsdaten einliest, den Zustandsautomaten in ein Programm übersetzt und das mindestens eine Instrument gemäß dem Steuerprogramm steuert. Weiterhin ist eine Vermittlungseinrichtung vorgesehen, die wenigstens einen während der Ausführung des Steuerprogramms empfangenen Einstellwert einer realen oder einer virtuellen Einstelleinrichtung zu mindestens einer mathematischen Funktion zuordnet, durch die ein Skalierfaktor für einen bestimmten Stellwert erhalten wird. Die Versorgungseinrichtung zeichnet sich dadurch aus, dass die Vermittlungseinrichtung eine Liste mit mehreren Vermittlern aufweist.

Ein wesentlicher Gedanke der vorliegenden Erfindung liegt also darin, dass die Versorgungseinrichtung eine Vermittlungseinrichtung umfasst, die einen empfangenen Einstellwert einer bestimmten mathematischen Funktion zuordnet, wobei die mathematische Funktion dem Einstellwert einen Skalierfaktor zuordnet, der wiederum zur Skalierung eines bestimmten Stellwerts dient. Auf diese Weise ist es möglich, dass beispielsweise bei der Einstellung eines Parameters durch einen Benutzer durch Betätigung einer realen Einstelleinrichtung, wie beispielsweise eines Potentiometers an einem Chirurgiegerät, eine Veränderung von mindestens einem Stellwert, insbesondere jedoch von mehreren Stellwerten bewirkt wird. Zur Entwurfszeit des Steuerprogramms werden also Regeln definiert, wie ein oder mehrere Einstelleinrichtungen (Potentiometer oder dergleichen) die Stellwerte des Steuerprogramms skalieren, d.h. verändern. Die mathematischen Skalierfunktionen erzeugen Kennlinien, die eine vorgenommene Einstellung mehreren unterschiedlichen Skalierfaktoren für mehrere Stellwerte zuordnen können. Ein und dieselbe Einstellung bzw. ein und derselbe Einstellwert kann folglich unterschiedliche Skalierfaktoren für unterschiedliche Stellwerte erzeugen. Weiterhin ist es durch die Erfindung möglich, dass beim Ausführen eines Sprungs bzw. eines Zustandsübergangs zwischen zwei Zuständen des Zustandsautomaten aufgrund einer wahren Zustandsübergangsregel eine virtuelle oder auch eine reale Einstelleinrichtung aufgrund einer einfachen Regel und einem normierten Messwert oder einer Konstanten verändert wird. Dabei wird die Regel und der normierte Messwert oder die Konstante vorzugsweise zur Entwurfszeit der Steuertabelle bzw. des Zustandsautomaten festgelegt.

Eine reale Einstelleinrichtung ist vorzugsweise durch den Benutzer eines Chirurgiegeräts betätigbar und wird durch einen Einstellknopf oder dergleichen Einstellelement gebildet. Eine virtuelle Einstelleinrichtung ist hingegen kein manuell betätigbares Einstellelement, sondern wird vielmehr durch mindestens eine Stellregel für mindestens eine Transition, d.h. mindestens einen Zustandsübergang zwischen zwei Zuständen des Zustandsautomaten, gebildet. Das Resultat der Stellregel der virtuellen Einstelleinrichtung bildet dabei den Einstellwert der virtuellen Einstelleinrichtung, und entspricht folglich einem Einstellwert, der durch einen Benutzer an einer realen Einstelleinrichtung festgelegt werden kann. In beiden Fällen ist entscheidend, dass der jeweilige erhaltene Einstellwert einer bestimmten Funktion durch die Vermittlungseinrichtung zugeordnet wird, die wiederum den Skalierfaktor für einen bestimmten Stellwert erzeugt. Aus Sicherheitsgründen lassen sich die Stellwerte durch die Skalierfaktoren vorteilhafter Weise nur verkleinern, weshalb die Skalierfaktoren in diesem Fall kleiner als 1 sein müssen. Der Skalierfaktor dient folglich zur Anpassung und insbesondere zur Verkleinerung eines beliebigen Stellwerts, wie beispielsweise einer Spannung, einer Leistung, einer Zeitspanne oder dergleichen. Bei dem Stellwert kann es sich um jeden erdenklichen einstellbaren Parameter des Zustandsautomaten handeln. Der Skalierfaktor wird mit dem jeweiligen zugeordneten Stellwert multipliziert und resultiert so in einem einzustellenden Stellwert, der von der Steuerung vorgegeben wird.

Vorzugsweise sind einem Einstellwert einer Einstelleinrichtung durch die Vermittlungseinrichtung mehrere mathematische Funktionen zugeordnet, die zur Erzeugung von Skalierfaktoren für die Anpassung unterschiedlicher Stellwerte vorgesehen sind. Durch die Verwendung unterschiedlicher mathematischer Funktionen ist es auf diese Weise möglich, denselben Einstellwert zur Erzeugung von unterschiedlichen Skalierfaktoren einzusetzen.

Die Einstellwerte der realen bzw. virtuellen Einstelleinrichtung weisen vorzugsweise einen Wert zwischen 0 und 1 auf, sind also auf den Bereich zwischen 0 und 1 normiert. Dadurch lassen sich die Werte sämtlicher Einstelleinrichtungen einheitlich und einfach durch die mathematischen Funktionen verarbeiten.

Eine Vermittlungseinrichtung der hier beanspruchten Art ist vorzugsweise bereits in der Steuertabelle des Zustandsautomaten realisiert und wird durch die Steuereinheit zusammen mit dem Zustandsautomaten in das Steuerprogramm übersetzt. Die Vermittlungseinrichtung weist mehrere Vermittler nach Art einer Liste auf, wobei jeder Vermittler einen Einstellwert einer Einstelleinrichtung zu einer einzigen mathematischen Funktion zuordnet, während ein weiterer Vermittler der Vermittlungseinrichtung einen Einstellwert derselben Einstelleinrichtung zu einer anderen mathematischen Funktion zuordnen kann. Auf diese Weise ist es möglich, einen Einstellwert einer bestimmten Einstelleinrichtung zu mehreren unterschiedlichen mathematischen Funktionen zuzuordnen und folglich unterschiedliche Skalierfaktoren für unterschiedliche Stellwerte auf der Grundlage ein und desselben Einstellwerts einer realen oder virtuellen Einstelleinrichtung zu erzeugen.

Zur Lösung der oben genannten Aufgabe wird auch ein Verfahren zur Erzeugung eines Steuerprogramms mit den Merkmalen des Anspruchs 8 vorgeschlagen. Das Verfahren dient wie gesagt zur Erzeugung eines Steuerprogramms für eine Versorgungseinrichtung mindestens eines chirurgischen Instruments, insbesondere für eine Versorgungseinrichtung nach einem der vorhergehenden Ansprüche, wobei das Verfahren die Schritte umfasst: Einlesen von Konfigurationsdaten, die einen Zustandsautomaten mit einer Vielzahl von Zuständen beschreiben; Übersetzen des Zustandsautomaten mit einer Vielzahl von Zuständen in ein Steuerprogramm durch eine Steuereinheit; Steuern des mindestens einen chirurgischen Instruments gemäß dem Steuerprogramm durch die Steuereinheit. Erfindungsgemäß umfasst das Verfahren die weiteren Schritte: Empfangen von mindestens einem Einstellwert einer realen oder virtuellen Einstelleinrichtung; Berechnen mindestens eines Skalierfaktors mittels des Einstellwerts und mindestens einer zugeordneten mathematischen Funktion, und Ändern mindestens eines zugeordneten Stellwertes auf der Grundlage des Skalierfaktors. Das Verfahren zeichnet sich dadurch aus, dass die Steuereinheit nach dem Empfang eines Einstellwertes nach mindestens einer zugeordneten Funktion sucht.

Die Berechnung des Skalierfaktors bzw. die Multiplikation des berechneten Skalierfaktors mit einem Stellwert erfolgt dann vorzugsweise bei einem Zustandsübergang zwischen zwei Zuständen des Zustandsautomaten, bei dem die Steuertabelle durch die Steuereinheit neu berechnet wird. Denkbar sind jedoch grundsätzlich auch andere Realisierungen zur Berechnung des neuen Stellwerts.

Die Erfindung wird im Folgenden anhand der Zeichnung näher erläutert. Es zeigen:
- Fig. 1: eine Versorgungseinrichtung gemäß der Erfindung mit einem beispielhaften angeschlossenen elektrochirurgischen Instrument, und
- Fig. 2: eine schematische Darstellung einzelner Komponenten der Versorgungseinrichtung gemäß der Erfindung.

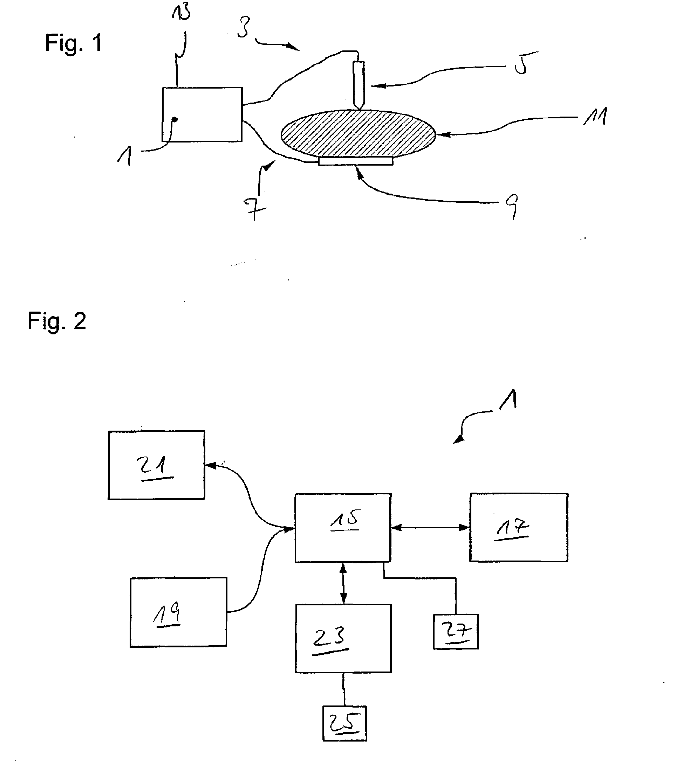

Die Fig. 1 zeigt eine Versorgungseinrichtung 1, die über eine erste HF-Leitung 3 mit einem elektrochirurgischen Instrument 5 verbunden ist. Eine zweite HF-Leitung 7 führt zu einer Neutralelektrode 9. Zum Anlegen einer HF-Spannung U, die von der Versorgungseinrichtung 1 bereitgestellt wird, umfasst das Instrument 5 eine aktive Elektrode. Somit kann die HF-Spannung U zwischen der Neutralelektrode 9 und der aktiven Elektrode angelegt werden.

Wie in der Fig. 1 gezeigt ist, können die Neutralelektrode 9 und die aktive Elektrode des elektrochirurgischen Instruments 5 verwendet werden, um einen HF-Strom I in ein biologisches Gewebe einzubringen. Die Fig. 1 zeigt einen Torso 11, an dem die selbstklebende Neutralelektrode 9 angebracht ist. Die aktive Elektrode des elektrochirurgischen Instruments 5 wird dazu verwendet, das Gewebe auf der anderen Seite des Torsos 11 zu schneiden oder zu koagulieren. Bei dem in der Fig. 1 gezeigten Instrument handelt es sich rein beispielhaft um ein monopolares Instrument, welches also nur eine aktive Elektrode aufweist. Statt der Neutralelektrode 9 kann jedoch auch ein bipolares Instrument mit zwei aktiven Elektroden vorgesehen sein. Die Funktion der Versorgungseinrichtung 1 gemäß der Erfindung wird hier rein beispielhaft anhand eines elektrochirurgischen Instruments erläutert. Es versteht sich, dass es sich auch um einen gänzlich anderen Instrumententyp handeln kann, wie beispielsweise ein wasserstrahlchirurgisches Instrument oder dergleichen.

Die Versorgungseinrichtung 1 bildet üblicherweise einen Teil eines Chirurgiegeräts 13, welches normalerweise Einstelleinrichtungen umfasst, die durch einen Benutzer der Chirurgieeinrichtung manuell betätigbar sind.

Wie in Fig. 2 gezeigt ist, umfasst die Versorgungseinrichtung 1 eine Steuereinheit 15 und einen HF-Generator 17. Der HF-Generator 17 dient dazu, eine geeignete HF-Spannung U für das elektrochirurgische Instrument 5 und die Neutralelektrode 9 bereitzustellen. Die Steuereinheit 15 steuert den HF-Generator 17, indem sie Steuersignale an diesen ausgibt. Darüber hinaus empfängt die Steuereinheit 15 von dem HF-Generator 17 Sensorsignale, die Auskünfte über den Zustand des HF-Generators 17 sowie über den applizierten HF-Strom I bzw. die HF-Spannung U geben. Weiterhin kann die Steuereinheit 15 Sensorsignale beispielsweise bezüglich der Gewebeimpedanz R, einer HF-Leistung P oder einem Wirkleistungsfaktor cos Φ empfangen.

Die Steuereinheit 15 ist vorzugsweise dazu ausgebildet, mehrere unterschiedliche Betriebsmodi bereitzustellen, die sich beispielsweise auf die zu applizierende HF-Spannung U oder HF-Leistung P auswirken. So kann die Steuereinheit einen Modus zum Koagulieren und einen weiteren Modus zum Schneiden von Gewebe anbieten. Des Weiteren können weitere Modi für unterschiedliche elektrochirurgische Instrumente 5, beispielsweise für monopolare oder biplare Instrumente, oder für unterschiedliche Gewebetypen (Leber- oder Muskelgewebe) bereitgestellt werden.

Die Versorgungseinrichtung 1 umfasst eine Eingabeeinheit 19, die es ermöglicht, Eingaben von einem Benutzer des elektrochirurgischen Instruments 5 zu empfangen. Die Eingabeeinheit 19 ist vorzugsweise als reale Einstelleinrichtung in Form von Dreh- oder Druckknöpfen an dem Chirurgiegerät 13 zur Betätigung durch einen Benutzer realisiert. Der Benutzer kann also über die Eingabeeinheit 19 einen bestimmten Betriebsmodus auswählen und die Steuereinheit 15 derart aktivieren, dass der HF-Generator 17 und somit das elektrochirurgische Instrument 5 in diesem Betriebsmodus betrieben werden. Weiterhin kann der Benutzer mittels der Eingabeeinheit 19 Einstellungen, beispielsweise zur Veränderung der Schnittleistung, vornehmen. Hierzu können ein oder mehrere reale Einstelleinrichtungen an dem Chirurgiegerät 13 vorgesehen sein. Sofern ein Benutzer der Versorgungseinrichtung bei einer aktiven Steuerung eine Veränderung eines Parameters, wie beispielsweise der HF-Spannung, der HF-Leistung, einer Zeitspanne oder dergleichen wünscht, stellt er mittels der Einstelleinrichtung einen bestimmten Einstellwert ein.

Um die Auswahl eines bestimmten Betriebsmodus zu erleichtern und die Statusinformationen bezüglich des HF-Generators 17 und/oder der Steuereinheit 15 und/oder des elektrochirurgischen Instruments 5 anzuzeigen, umfasst die Versorgungseinrichtung 1 des Weiteren eine Ausgabeeinheit 21, die vorzugsweise einen Bildschirm zum Anzeigen von Informationen umfasst.

Die Steuereinheit 15 steht in kommunikativer Verbindung mit einer Speichereinheit 23, die über eine Schnittstelle 25 verfügt. Über die Schnittstelle 25 können Konfigurationsdaten in die Speichereinheit 23 geladen werden. Die Steuereinheit 15 hat eine Zeiterfassungseinheit 27, die Sensorsignale in Form von Zeitsignalen vorgibt, die das Erfassen von vorgegebenen Zeitintervallen ermöglichen.

Die Speichereinheit 23 umfasst ein Rahmenprogramm, das durch die Steuereinheit 15 ausgeführt wird. Dieses Rahmenprogramm veranlasst die Steuereinheit 15, Konfigurationsdaten aus der Speichereinheit 23 zu laden und ein Steuerprogramm zu generieren, das die Steuerung des an die Versorgungseinrichtung 1 angeschlossenen Instruments 5 ermöglicht. Vorzugsweise wird hierbei ein tabellengesteuertes Schaltwerk implementiert, das eine Vielzahl von Zuständen Z0 bis Zx hat, wobei die einzelnen Zustände Z0 bis Zx in Abhängigkeit von vorgegebenen Randbedingungen eingenommen werden.

Die in der Speichereinheit 23 gespeicherten Konfigurationsdaten modellieren vorzugsweise tabellarisch einen endlichen Automaten bzw. einen Zustandsautomaten. Ein Zustandsautomat umfasst mehrere Zustände Z0 bis Zx, wobei ein Übergang zwischen den einzelnen Zuständen durch Transitionen erfolgt, die einen Übergang zwischen einem ersten und einem zweiten Zustand beschreiben. Jeder Transition ist wenigstens eine Transitionsregel, auch Zustandsübergangsregel genannt, zugeordnet, die wenigstens eine Bedingung enthält, bei deren Erfüllung die Steuereinheit 15 aus einem Steuermodus gemäß dem ersten Zustand in einen Steuermodus gemäß dem zweiten Zustand wechselt. Eine Transitionsregel kann beispielsweise die Bedingung umfassen, dass ein Übergang in einen anderen Zustand nur dann vorgenommen wird, wenn die Impedanz R größer als beispielsweise 80 Ohm oder der Wirkleistungsfaktor cos Φ größer als 0,5 ist. Weiterhin sind Transitionsregeln denkbar, bei denen eine Bedingung über einen vorgegebenen Zeitraum eingehalten werden muss. Darüber hinaus sind Transitionsregeln denkbar, die nur dann vorgenommen werden, wenn der Wirkleistungsfaktor cos Φ für mehr als 5 Millisekunden größer als 0,5 ist.

Gemäß der vorliegenden Erfindung umfasst die Versorgungseinrichtung 1 eine Vermittlungseinrichtung, die wenigstens einen während der Ausführung des Steuerprogramms in der Steuereinheit 15 empfangenen Einstellwert einer realen oder einer virtuellen Einstelleinrichtung zu mindestens einer mathematischen Funktion zuordnet. Die Vermittlungseinrichtung entspricht quasi einer Zuordnungseinrichtung, die in der Steuertabelle bzw. in dem Zustandsautomaten in der Speichereinheit 23 implementiert ist und einer Einstelleinrichtung ein oder mehrere unterschiedliche mathematische Funktionen zuordnet.

Anhand des folgenden Beispiels wird die Funktion der Vermittlungseinrichtung 1 für den Fall erläutert, dass ein Benutzer einen Einstellwert mittels einer realen Einstelleinrichtung, also beispielsweise eines Dreh- oder Druckknopfes eines Chirurgiegeräts, während die Steuerung den Zustandsautomaten ausführt, manuell verändert:
Im vorliegenden Beispielfall wird angenommen, dass ein Benutzer des Chirurgiegeräts bzw. des elektrochirurgischen Instruments gemäß Fig. 1 eine Veränderung der Schnittkraft vornehmen möchte. Aus Sicherheitsgründen ist jedoch vorzugsweise nur eine Verminderung der Schnittkraft möglich. Vorzugsweise sind alle einstellbaren Werte einer Einstelleinrichtung zur Vereinfachung auf Werte zwischen 0 und 1 normiert. Dabei entspricht der Wert 1 vorzugsweise dem maximal einstellbaren Einstellwert der Einstelleinrichtung.

Beispielsweise weist eine Einstelleinrichtung X1 den anfänglichen Einstellwert x1=0,7 auf und ein Benutzer ändert den Einstellwert der Einstelleinrichtung x1 auf 0,6. Nach der Veränderung des Einstellwerts meldet die reale Einstelleinrichtung also einen Einstellwert von x1=0,6. Sobald die Einstelleinrichtung einen Einstellwert von x1=0,6 meldet, sucht die Steuerung nach Vermittlern in der Vermittlungseinrichtung, die der Einstelleinrichtung X1 zugeordnet sind. Beispielsweise ist es denkbar, dass die Einstelleinrichtung X1 durch zwei verschiedene Vermittler V1 und V4 referenziert wird. Die beiden Vermittler V1 und V4 ordnen dann den Einstellwert x1=0,6 jeweils zwei unterschiedlichen mathematischen Funktionen 1 und 2 zu, wie im Folgenden beispielhaft dargestellt ist:

| | |
|---|---|
| Vermittler V1 | ⇒Mathe. Funktion 1: y=Ax²+Bx+C; Parameter: A=0,5; B=0,5; C=0 Ziel: Zustand Z1; Einstellung 1: U_HFmax → Skalierungsfaktor des Stellwertes Z1.U_HFmax wird auf 0,68 gestellt |
| Vermittler V4 | ⇒Mathe. Funktion 2: y=Ae^{BX}+C; Parameter: A=0,3; B=1; C=0 Ziel: Sprung 2A; Wartezeit → Skalierungsfaktor des Stellwertes Trans 21.TV wird auf 0,55 gestellt |

Es wird also deutlich, dass die Vermittlungseinrichtung im Prinzip eine Liste von mehreren Vermittlern V1 bis Vx umfasst, die jeweils eine Einstelleinrichtung zu einer mathematischen Funktion zuordnen. Mehrere Vermittler können derselben Einstelleinrichtung auch mehrere Funktionen zuordnen, wie unten anhand der Vermittler V1 und V2 gezeigt ist, die beide eine Zuordnung der Einstelleinrichtung X1 vornehmen, jedoch zu unterschiedlichen Funktionen. Im Folgenden wird das Funktionsprinzip der Vermittlungseinrichtung zur Verdeutlichung wiedergegeben:

| | | | |
|---|---|---|---|
| V1: | X1 → | F1(x1) → | Y1 |
| V2: | X1 → | F2(x1) → | Y2 |
| V3: | X2 → | F3(x2) → | Y3 |

| | |
|---|---|
| U_{Regler} | = Y1 * U_{Soll} |
| P_{Regler} | = Y2 * P_{Soll} |
| R_{Schwelle} | = Y3* R_{SchwelleOrg} |

Es wird deutlich, dass beispielsweise der Vermittler V1, der Einstelleinrichtung X1 bzw. einem Einstellwert x1 der Einstelleinrichtung X1 eine Funktion F1 zuordnet und ein weiterer Vermittler V2 der Vermittlungseinrichtung derselben Einstelleinrichtung X1 eine zweite unterschiedliche Funktion F2 zuordnen kann. Ein dritter Vermittler V3 kann beispielsweise einer weiteren Einstelleinrichtung X2 eine dritte unterschiedliche Funktion F3, aber auch eine der Funktionen F1 oder F2 oder eine beliebige andere Funktion zuordnen. Entscheidend ist, dass durch die Möglichkeit der Zuordnung einer Einstelleinrichtung zu mehreren unterschiedlichen mathematischen Funktionen durch die Vermittlungseinrichtung mehrere Skalierfaktoren Y1 bis Yx durch die Anwendung der Einstellwerte auf die jeweiligen Funktionen erhalten werden. Anders ausgedrückt wird ein Skalierfaktor Y dadurch erhalten, dass ein Einstellwert x in eine mathematische Funktion F eingesetzt wird, wobei die Verknüpfung zwischen dem Einstellwert und der richtigen Funktion durch einen Vermittler festgelegt ist. Damit aus Sicherheitsgründen nur eine Verkleinerung bestehender Stellwerte möglich ist, ist die Funktion vorzugsweise so gewählt, dass der resultierende Skalierfaktor einen Wert zwischen 0 und 1 annimmt.

Die erhaltenen Skalierfaktoren Y1 bis Y3 können dann auf unterschiedliche, frei wählbare Stellwerte angewendet werden, wie oben anhand der Stellwerte U (HF-Spannung), P (HF-Leistung) und R (Widerstand) beispielhaft dargelegt ist. Mit anderen Worten werden die erhaltenen Skalierfaktoren mit Stellwerten multipliziert, wobei das Ergebnis den durch die Steuereinheit vorzugebenden endgültigen Stellwert bildet.

Vorzugsweise erfolgt eine Aktualisierung der Skalierfaktoren Y1 bis Y3 während eines Zustandsübergangs von einem Zustand in einen anderen Zustand des Zustandsautomaten. Wird also während des Ablaufs des Steuerprogramms eine Veränderung eines Einstellwertes vorgenommen, wird der nächste Zustandsübergang des Zustandsautomaten abgewartet, weil dann die Steuertabelle durch die Steuereinheit neu berechnet wird. Während des Zustandsübergangs wird dann der Einstellwert in die zugeordnete Funktion eingesetzt und der resultierende Skalierfaktor berechnet. Der Skalierfaktor wird dann mit einem zugeordneten Stellwert multipliziert, wodurch schließlich der einzustellende endgültige Stellwert resultiert.

Bei den mathematischen Funktionen kann es sich um beliebige, beispielsweise um quadratische Funktionen oder um Exponentialfunktionen handeln. Entscheidend ist, dass ein und derselbe Einstellwert mehreren verschiedenen mathematischen Funktionen zugeordnet sein kann, die dem Einstellwert folglich verschiedene Skalierfaktoren zuordnen. In dem oben angegebenen Beispiel wird deutlich, dass auf diese Weise derselbe Einstellwert x1=0,6 zu unterschiedlichen Skalierfaktoren für eine

Spannung und für eine Wartezeit führen kann, nämlich beispielsweise zu einem Skalierfaktor von 0,68 für die Spannung U_HFmax und zu einem Skalierfaktor von 0,55 für die Wartezeit Trans 21.TV. Indem der Einstellwert x1 in die Funktion 1 und in die Funktion 2 eingesetzt wird, werden also zwei unterschiedliche Skalierfaktoren Y1 und Y2 erhalten. Das "Ziel", mit dem ein Skalierfaktor letztendlich multipliziert werden soll, ist ein bestimmter zugeordneter Stellwert, der innerhalb der Grundeinstellung des Zustandsautomaten und/oder in den Zuständen des Zustandsautomaten und/oder in den Sprungbedingungen zwischen einzelnen Zuständen frei definiert werden kann.

Ein Einstellwert kann jedoch nicht nur durch eine manuelle Betätigung einer realen Einstelleinrichtung durch einen Benutzer verändert werden. Vielmehr kann sich ein neuer Einstellwert einer virtuellen oder realen Einstelleinrichtung auch aus einem Gewebeparameter und/oder einem HF-Messwert und generell aus jedem Sensorsignal ergeben, welches von der Steuereinheit 15 empfangen oder von dieser generiert wird, und die als Parameter für Transitions- bzw. Zustandsübergangsregeln bei einem Sprung von einem Zustand in einen anderen Zustand des Zustandsautomaten dienen. In diesem Fall ist also alternativ oder zusätzlich zu der mindestens einen realen Einstelleinrichtung quasi eine virtuelle Einstelleinrichtung vorgesehen, die im Prinzip mindestens eine einfache Stellregel für einen erfassten Messwert oder dergleichen umfasst, wobei die Ergebnisse der Stellregeln vorzugsweise wiederum auf Werte zwischen 0 und 1 normiert werden. Die Ergebnisse der Stellregeln bilden folglich die Einstellwerte der virtuellen Einstelleinrichtung, wobei die Einstellwerte wiederum unterschiedlichen realen oder virtuellen Einstelleinrichtungen zugeordnet sein können. Die Stellregeln werden zur Entwurfszeit des Zustandsautomaten festgelegt und können beispielsweise wie folgt aussehen:
Initialisiere x4=0
Initialisiere x5=0,3
Wenn Sprung 1A, dann stelle x4=2000 mA/IHFrms (Normierung auf 0...1)
Wenn Sprung 1B, dann stelle x4=x4+0,2
Wenn Sprung 1C, dann stelle x4=0,3
Wenn Sprung 2A, dann stelle x5=x5*1,05
Wenn Sprung 3A, dann stelle x1=x1*0,2.

Bei den Sprüngen 1A, 1B etc. handelt es sich um Transitionsregeln, die, sofern sie erfüllt werden, einerseits eine Aktion auslösen, insbesondere also einen Sprung von einem ersten Zustand in einen zweiten Zustand, die aber andererseits auch eine zugeordnete Stellregel (s.o.) auslösen. Beispielsweise kann ein Zustand 1 des Zustandsautomaten wie folgt definiert sein:
Zustand 1: Start
   -> U_HFmax := 200Vpeak
   -> 1_Hfmax : = 3A rms
   -> P_HFmax 120W
Sprung 1A>>> Widerstand überschritten< < <
Sprung 1A: Wenn 1 mal RLast >= 80 Ohm, dann wechsle zu Zustand: 3
Sprung 1B>>> cos Φ < 0,5 (=LF 16384) <<<
Sprung 1B: Wenn 50 mal LF < 16384 , dann wechsle zu Zustand: 3
Sprung 1C>>> Zeitlimit <<<
Sprung 1C: Wenn 10000 mal, dann wechsle zu Zustand: 4

Ist beispielsweise die Transitionsregel 1A bzw. der Sprung 1A erfüllt, das heißt also, dass der Widerstand überschritten wurde, wechselt die Steuerung in den Zustand 3. Die wahre Transitionsregel 1A löst gemäß der Erfindung aber nicht nur einen Zustandsübergang aus, sondern führt gleichzeitig zu einer Änderung eines zugeordneten Einstellwertes. Wenn also die Transitionsregel 1A erfüllt wird, kann beispielsweise eine Stellregel in der virtuellen Einstelleinrichtung, wie oben gezeigt, so definiert sein, dass eine Einstelleinrichtung X4 auf den Einstellwert x4 = 2000 mA/IHFrms eingestellt wird, wobei wiederum eine Normierung des resultierenden Einstellwertes x4 auf einen Wert zwischen 0 und 1 erfolgt. Ist hingegen die Transitionsregel 1B erfüllt, d.h. der Wirkleistungsfaktor cos Φ ist kleiner als 0,5, dann kann eine Stellregel der virtuellen Einstelleinrichtung einen Einstellwert von x4 = x4 + 0,2 der Einstelleinrichtung X4 bewirken.

Auf diese Weise lassen sich verschiedenen Transitionsregeln unterschiedliche Stellregeln zuordnen, die im Falle einer Erfüllung der Transitionsregeln eine Veränderung eines Einstellwertes einer virtuellen oder einer realen Einstelleinrichtung bewirken. Vorzugsweise ist eine einzige Transitionsregel einer einzigen Stellregel der virtuellen Einstelleinrichtung zugeordnet. Es kann jedoch auch vorgesehen sein, dass eine Transitionsregel mehreren Stellregeln zugeordnet ist und folglich eine Veränderung von mehreren Einstellwerten unterschiedlicher Einstelleinrichtungen bewirkt.

Die Berechnung der Skalierfaktoren auf der Grundlage der neuen Einstellwerte und der verschiedenen mathematischen Funktionen erfolgt dann wie oben für den Fall einer realen Einstelleinrichtung beschrieben mittels der Zuordnung durch Vermittler.

Das Grundprinzip, welches der vorliegenden Erfindung zu Grunde liegt, soll anhand der nachfolgenden Tabelle noch einmal verdeutlicht werden.

| Einstellwert = x = [0...1] | Funktion = y = [0...1] | Ziel |
|---|---|---|
| reale Einstelleinrichtung (z.B. Potentiometer o.ä.) | y= Ax²+Bx+C | U_{Regler} = y * U_{Soll} |
| R/R_{N} | . | P_{Regler} = y * P_{Soll} |
| .. | . | R_{Schwelle} = y* R_{SchwelleOrg} |
| . | y=A*e^{BX}+C | |
| U/U_{N} | | |
| | y = [0...1] | |

Insgesamt zeigt sich somit, dass durch die vorliegende Erfindung während der Ausführung des Zustandsautomaten in der Steuereinheit 15 zum einen durch einen Benutzer dynamisch auf Stellwerte des Zustandsautomaten Einfluss genommen werden kann, und dass zum anderen Messwerte oder andere Parameter, die für Transitionsregeln herangezogen werden, eine Veränderung von Einstellwerten durch Stellregeln bewirkt werden können.

Entscheidend ist insgesamt, dass die Vermittlungseinrichtung eine Zuordnung eines Einstellwertes einer realen bzw. einer virtuellen Einstelleinrichtung zu einer mathematischen Funktion vornimmt, durch die ein Skalierfaktor erhalten wird, der die Skalierung bzw. Veränderung und insbesondere die Verkleinerung eines Stellwerts des Zustandsautomaten bewirkt. Unter Einstelleinrichtung ist dabei eine Einrichtung zu verstehen, die entweder durch eine manuelle Maßnahme durch einen Benutzer oder durch ein oder mehrere Stellregeln eine Veränderung von Einstellwerten bewirken kann. Die Einstelleinrichtung kann folglich ein "Hardware-Element" oder ein "Software-Element" der Versorgungseinrichtung sein. Entscheidend ist, dass sie auf eine bestimmte Aktion hin eine Veränderung von mindestens einem Einstellwert vornimmt.

### Bezugszeichenliste

- 1: Versorgungseinrichtung
- 3: erste HF-Leitung
- 5: chirurgisches Instrument
- 7: zweite HF-Leitung
- 9: Neutralelektrode
- 11: Torso
- 13: Chirurgiegerät
- 15: Steuereinheit
- 17: HF-Generator
- 19: Eingabeeinheit
- 21: Ausgabeeinheit
- 23: Speichereinheit
- 25: Schnittstelle
- 27: Zeiterfassungseinheit

## Patentansprüche

1. Versorgungseinrichtung für ein Chirurgiegerät zum Betreiben mindestens eines chirurgischen Instruments (5), insbesondere eines elektrochirurgischen Instruments und/oder eines kryochirurgischen Instruments und/oder wasserstrahlchirurgischen Instruments, umfassend:
- eine Steuereinheit (15) zur Steuerung des mindestens einen Instruments (5),
- eine Speichereinheit (23) zum Speichern von Konfigurationsdaten, insbesondere Stellwerte, die einen Zustandsautomaten mit einer Vielzahl von Zuständen beschreiben,
wobei die Steuereinheit (15) derart ausgebildet ist, dass sie die Konfigurationsdaten einliest, den Zustandsautomaten in ein Steuerprogramm übersetzt und das mindestens eine Instrument (5) gemäß dem Steuerprogramm steuert, wobei eine Vermittlungseinrichtung vorgesehen ist, die wenigstens einen während der Ausführung des Steuerprogramms empfangenen Einstellwert einer realen oder einer virtuellen Einstelleinrichtung zu mindestens einer mathematischen Funktion zuordnet, durch die ein Skalierfaktor für einen Stellwert erhalten wird,
**dadurch gekennzeichnet,dass**
die Vermittlungseinrichtung eine Liste mit mehreren Vermittlern umfasst.

2. Versorgungseinrichtung nach Anspruch 1,
**dadurch gekennzeichnet,dass**
die reale Einstelleinrichtung durch den Benutzer eines Chirurgiegeräts betätigbar ist.

3. Versorgungseinrichtung nach Anspruch 1 oder 2,
**dadurch gekennzeichnet,dass**
die virtuelle Einstelleinrichtung mindestens eine Stellregel für mindestens eine Transition zwischen zwei Zuständen des Zustandsautomaten umfasst.

4. Versorgungseinrichtung nach Anspruch 2,
**dadurch gekennzeichnet,dass**
das Resultat der Stellregel den Einstellwert der virtuellen Einstelleinrichtung bildet.

5. Versorgungseinrichtung nach einem der vorhergehenden Ansprüche,
**dadurch gekennzeichnet, dass**
der Skalierfaktor zur Anpassung, insbesondere zur Verkleinerung des Stellwerts dient.

6. Versorgungseinrichtung nach einem der vorhergehenden Ansprüche,
**dadurch gekennzeichnet,dass**
einem Einstellwert durch die Vermittlungseinrichtung mehrere mathematische Funktionen zugeordnet sind, die zur Erzeugung von Skalierfaktoren für die Anpassung unterschiedlicher Stellwerte vorgesehen sind.

7. Versorgungseinrichtung nach einem der vorhergehenden Ansprüche,
**dadurch gekennzeichnet,dass**
der Einstellwert der realen bzw. virtuellen Einstelleinrichtung zwischen 0 und 1 liegt.

8. Verfahren zur Erzeugung eines Steuerprogramms für eine Versorgungseinrichtung (30) mindestens eines chirurgischen Instruments (5) nach einem der vorhergehenden Ansprüche, wobei das Verfahren die Schritte umfasst:
- Einlesen von Konfigurationsdaten, die einen Zustandsautomaten mit einer Vielzahl von Zuständen beschreiben;
- Übersetzen des Zustandsautomaten mit der Vielzahl von Zuständen in ein Steuerprogramm durch eine Steuereinheit (15);
- Steuern des mindestens einen chirurgischen Instruments (5) gemäß dem Steuerprogramm durch die Steuereinheit (15);
- Empfangen von mindestens einem Einstellwert einer realen oder virtuellen Einstelleinrichtung;
- Berechnen mindestens eines Skalierfaktors mittels des Einstellwertes und mindestens einer zugeordneten mathematischen Funktion;
- Ändern mindestens eines zugeordneten Stellwertes auf der Grundlage des Skalierfaktors,
**dadurch gekennzeichnet,dass**
die Steuereinheit nach dem Empfang eines Einstellwertes nach mindestens einer zugeordneten Funktion in einer Liste von Vermittlern sucht.

9. Verfahren nach Anspruch 8,
**dadurch gekennzeichnet,dass**
die Aktualisierung von Skalierfaktoren während einer Transition von einem Zustand in einen anderen Zustand des Zustandsautomaten erfolgt.

## Claims

1. Supply device for a surgical apparatus for operating at least one surgical instrument (5), in particular an electrosurgical instrument and/or a cryosurgical instrument and/or water jet surgical instrument, comprising:
- a control unit (15) for controlling the at least one instrument (5),
- a memory unit (23) for storing configuration data, in particular manipulated variables which describe an automatic state changing system with a multiplicity of states,
the control unit (15) being designed in such a way that it reads in the configuration data, transfers the automatic state changing system into a control program and controls the at least one instrument (5) in accordance with the control program, a switching device being provided which assigns at least one setting value, received during the execution of the control program, of a real or a virtual setting device to at least one mathematical function by means of which a scale factor for a manipulated variable is obtained, **characterized in that** the switching device includes a list with a plurality of switches.

2. Supply device according to Claim 1, **characterized in that** the real setting device is operable by the user of a surgical device.

3. Supply unit according to Claim 1 or 2, **characterized in that** the virtual setting device includes at least one control rule for at least one transition between two states of the automatic state changing system.

4. Supply device according to Claim 2, **characterized in that** the result of the control rule forms the setting value of the virtual setting device.

5. Supply device according to one of the preceding claims, **characterized in that** the scaling factor is used to adapt, in particular to reduce the manipulated variable.

6. Supply device according to one of the preceding claims, **characterized in that** by means of the switching device a setting value is assigned a plurality of mathematical functions which are provided for generating scaling factors for adapting different manipulated variables.

7. Supply device according to one of the preceding claims, **characterized in that** the setting value of the real or virtual setting device lies between 0 and 1.

8. Method for producing a control program for a supply device (30) of at least one surgical instrument (5) according to one of the preceding claims, the method comprising the steps of:
- reading in configuration data which describe an automatic state changing system with a multiplicity of states;
- transferring the automatic state changing system with the multiplicity of states into a control program by means of a control unit (15);
- controlling the at least one surgical instrument (5) in accordance with the control program by means of the control unit (15);
- receiving at least one setting value of a real or virtual setting device;
- calculating at least one scaling factor by means of the setting value and at least one assigned mathematical function;
- changing at least one assigned manipulated variable on the basis of the scaling factor,
**characterized in that** after receiving a setting value the control unit searches in a list of switches for at least one assigned function.

9. Method according to Claim 8, **characterized in that** the scaling factors are updated during a transition from one state into another state of the automatic state changing system.

## Revendications

1. Dispositif d'alimentation d'appareil chirurgical destiné à alimenter au moins un instrument chirurgical (5), en particulier un instrument électrochirurgical, un instrument cryochirurgical et/ou un instrument chirurgical à jet d'eau, le dispositif d'alimentation comportant :
une unité de commande (15) qui commande le ou les instruments (5),
une unité de mémoire (23) qui conserve des données de configuration, en particulier des valeurs de réglage qui décrivent un automate d'état qui présente plusieurs états,
l'unité de commande (15) étant configurée de manière à lire les données de configuration, à transmettre les automates d'état dans un programme de commande et à commander le ou les instruments (5) selon un programme de commande,
un dispositif de transmission étant prévu pour associer au moins une valeur de réglage d'un dispositif de réglage réel ou virtuel, reçue pendant l'exécution du programme de commande, à au moins une fonction mathématique qui permet d'obtenir un facteur d'échelle pour une valeur de réglage,
**caractérisé en ce que**
le dispositif de transmission comporte une liste contenant plusieurs appareils de transmission.

2. Dispositif d'alimentation selon la revendication 1, **caractérisé en ce que** le dispositif de réglage réel peut être actionné par l'utilisateur de l'appareil chirurgical.

3. Dispositif d'alimentation selon les revendications 1 ou 2, **caractérisé en ce que** le dispositif de réglage virtuel comporte au moins une règle de réglage pour au moins une transition entre deux états de l'automate d'état.

4. Dispositif d'alimentation selon la revendication 2, **caractérisé en ce que** le résultat de la règle de réglage forme la valeur de réglage du dispositif virtuel de réglage.

5. Dispositif d'alimentation selon l'une des revendications précédentes, **caractérisé en ce que** le facteur d'échelle sert à adapter et en particulier à diminuer la valeur de réglage.

6. Dispositif d'alimentation selon l'une des revendications précédentes, **caractérisé en ce que** plusieurs fonctions mathématiques prévues pour former des facteurs d'échelle pour l'adaptation de différentes valeurs de réglage sont associées à une valeur de réglage par le dispositif de transmission.

7. Dispositif d'alimentation selon l'une des revendications précédentes, **caractérisé en ce que** la valeur de réglage du dispositif de réglage réel ou du dispositif de réglage virtuel est comprise entre 0 et 1.

8. Procédé de formation d'un programme de commande pour un dispositif d'alimentation (30) d'au moins un instrument chirurgical (5) selon l'une des revendications précédentes, le procédé comportant les étapes qui consistent à :
lire des données de configuration qui décrivent un automate d'état présentant plusieurs états,
faire traduire l'automate d'état qui présente plusieurs états en un programme de commande par une unité de commande (15),
faire commander le ou les instruments chirurgicaux (5) selon le programme de commande par l'unité de commande (15),
recevoir au moins une valeur de réglage d'un dispositif réel ou d'un dispositif virtuel de réglage,
calculer au moins un facteur d'échelle au moyen de la valeur de réglage et d'au moins une fonction mathématique associée,
modifier au moins une valeur de réglage associée sur la base du facteur d'échelle,
**caractérisé en ce que**
après réception d'une valeur de réglage, l'unité de commande cherche la ou les fonctions associées dans une liste de dispositifs de transmission.

9. Procédé selon la revendication 8, **caractérisé en ce que** l'actualisation de facteur d'échelle a lieu pendant la transition de l'automate d'état d'un état dans un autre état.
